# EUROPÄISCHE PATENTSCHRIFT

(11) **EP 1 524 109 B1**
(45) Veröffentlichungstag und Bekanntmachung des Hinweises auf die Patenterteilung: **04.12.2013**
(21) Anmeldenummer: 04023738.0
(22) Anmeldetag: 06.10.2004
(51) Int. Cl.: B32B 27/32, F16L 11/06

(54) **Verwendung eines PVC-freien Mehrschichtschlauchs mit verbesserter Peelsicherheit für medizinische Zwecke**
Use of a PVC-free multilayered tube with improved peelsecurity for medical purpose
Utilisation d'un tuyau multicouche a base de matière plastique, exempte de pvc avec résistance au pelage améliorée a usage médical

(30) Priorität: 17.10.2003 DE 10349011
(43) Veröffentlichungstag der Anmeldung: 20.04.2005
(73) Patentinhaber: Fresenius Medical Care Deutschland GmbH, 61352 Bad Homburg v.d.H. (DE)
(72) Erfinder: Gao, Xiaogang, Dr., 66606 St. Wendel (DE); Ahr, Uwe, 66606 St. Wendel (DE); Schmitt, Karl-Heinz, 66629 Freisen (DE)
(74) Vertreter: Mai, Dörr, Besier

(56) Entgegenhaltungen:
- EP-A- 0 765 740
- WO-A-95/13918
- WO-A-96/40512
- WO-A-98/00286
- WO-A-02/053359

## Beschreibung

Die Erfindung bezieht sich auf die Verwendung einer Pufferschicht in einem PVC-freien Mehrschichtschlauch für medizinische Zwecke mit verbesserter Sicherheit der Peelbarkeit eines knickschädigungsfreien Schlauchkringels umfassend den Mehrschichtschlauch gemäß Oberbegriff des Anspruchs 1.

Zum Stand der Technik werden die
WO-A-92/11820 (= D1),
EP-A-0765740 (= D2),
US-A-5,466,322 (= D3),
US-A-5,804,151 (= D4),
US-A-5,958,167 (= D5),
WO 98/00286 (= D6),
WO 02/053359 (= D7),
WO 96/40512 (= D8) und
WO 95/13918 (= D9) genannt.

PVC-freie Materialien (Non-PVC-Materialien) und daraus hergestellte einlagige Schläuche mit nur einer Schicht sind bspw. aus D1 bekannt. Hierin wird ein Schlauchmaterial für medizinische Zwecke vorgestellt, welches einen Blend aus Polyurethan Polyester aufweist, und das sowohl einer Sterilisationsbehandlung im Autoklaven unterzogen werden kann, heißsiegelfähig als auch mit hochfrequenter Energie siegel- und verschmelzbar ist.

D2 offenbart einen PVC-freien Mehrschichtschlauch für medizinische Zwecke mit wenigstens zwei Schichten, von denen eine Basisschicht A) aus einem ersten Kunststoffmaterial mit wenigstens einer Konnektionsschicht B) aus einem zweiten Kunststoffmaterial verbunden ist, wobei das erste Kunststoffmaterial wenigstens ein Polymer aufweist, das eine Hitzesterilisation bei ≥ 121 °C ohne Formschädigung übersteht, eine Härte Shore D ≤ 32 hat, das bei ≥ 121 °C eine Restspannung aufweist, die ausreicht, um an einer Konnektionsstelle einen Presssitz zu bilden, und aus dem ein Kringel oder eine Schlaufe mit einem Durchmesser von bis zu 60 mm ohne Knickschädigung geformt werden kann, während das zweite Kunststoffmaterial wenigstens ein Polymer aufweist, das während einer Hitzesterilisation bei 121 °C unter dem bei Ausbildung eines Presssitzes resultierenden Konnektionsdruck zum Fließen neigt und eine Härte Shore A) ≤ 65 aufweist, so dass die Formbeständigkeit des Kunststoffmaterials bei Temperaturen ≥ 121 °C gegeben ist und die des zweiten Kunststoffmaterials nicht mehr.
Es wird ferner in D2 ein Mehrschichtschlauch, der Schichten A), B) und C) enthält, beschrieben. Die Basisschicht A) ist 900-980 µm dick und besteht überwiegend aus SIS oder Polypropylen, die 10-50 µm dicke Konnektionsschicht B) besteht aus einem SEBS-Compound oder SEPS/SEP und die 10-50 µm dicke Deckschicht C) enthält PP-R. Die Abfolge der Schichten kann B)A)C)/C)A)B)/C)B)A)B) oder B)A)B)C), jeweils von innen nach außen, sein. Obwohl es mit dem Mehrschichtschlauch gemäß D2 gelingt, einen flexiblen Schlauch zur Verfügung zu stellen, der nach erfolgter Hitzesterilisation transparent ist, der ausreichende Knickstabilität aufweist und der mit Schlauchklemmen oder dergleichen abdichtbar ist und der darüber hinaus zusätzlich die Fähigkeit hat, mit einem Einbauteil, einen medizinischen Beutel oder einem Konnektor eine feste und dichte Verbindung einzugehen, und zwar äußerst einfach während einer möglichen Hitzesterilisationsbehandlung, kann der Schlauch der D2 beim Auseinanderziehen der Kringel beschädigt werden und ist somit immer noch verbesserungsbedürftig.

WO 98/00286 (=D6) beschreibt ein medizinisches Schlauchstück aus einer Außenschicht enthaltend ULDPE, einer Verbindungsschicht aus einem modifizierten Polyolefin und einer Innenschicht, die vorzugsweise PVC enthält.

Dokument WO 02/053359 (=D7) offenbart einen Mehrschichtschlauch mit einer Außenschicht, enthaltend 0 bis 60 % Polyolefin und 40 bis 100 % erstes thermoplastisches Polymer, einer Mittelschicht, enthaltend 35 bis 100 % zweites thermoplastisches Polymer und 0 bis 65 % zweites Polyolefin, und einer Innenschicht enthaltend 25 - 50 % drittes Polyolefin, 0 - 50 % viertes Polyolefin, 0 - 40 % RF siegelbares Polymer und 0 - 40 % drittes thermoplastisches Polymer. Die Mittelschicht kann gegebenenfalls vollständig aus SEBS bestehen, ist aber doppelt bis dreifach so dick wie die Außen- bzw. Innenschicht des Schlauches. Ein aus dem Mehrschichtschlauch gebildeter Schlauchkringel, sowie die Problematik der Peelbarkeit werden in D6 und D7 nicht beschrieben.

WO 96/40512 (= D8) und WO 95/13918 (= D9) beschreiben einen halogenfreien mehrschichtigen Polymerfilm, der für medizinische Zwecke verwendet werden kann. Der Polymerfilm kann aus 3 Schichten bestehen. Die "Skin-Layer" enthält ein Polypropylen-Copolymer, das 0-20 Gew. % SEBS enthalten kann, eine Kernschicht, enthaltend ein PP-Copolymer (45 Gew.%), ULDPE (40 Gew.%) und 15 Gew.% eines SEBS-Blockcopolymers, und eine RF-sensitive Schicht, die hauptsächlich ein PP-Copolymer und ULDPE enthält. Die Bildung von Schlauchkringeln wird nicht offenbart.

Medizinische Schläuche werden oft in Kringeln ausgeliefert. Auf diese Weise können Schläuche Platz sparend und ohne Verwirrungsgefahr transportiert werden. Schläuche können durch Fäden, Drähte oder Klammem in Kringeln zusammen gehalten werden. Besonders vorteilhaft ist es jedoch, wenn die Schläuche an ihrer Außenschicht so peelbar miteinander verschweißt sind, dass sie Kringel bilden. In dieser Ausführungsform kann der Kringel einfach auseinander gezogen werden, ohne dass vorher ein Halterungsmittel gelöst werden muss und als Abfall anfällt. Dies erleichtert auch die Handhabung. Die Herstellung solcher peelbar verschweißter Kringel gehört zum Stand der Technik (vgl. Dokumente D3 - D5).

Beim Auseinanderziehen der Kringel eines Schlauches gemäß D2 kann es nun vorkommen, dass sich der Schlauch nicht nur an der peelbaren Verschweißstelle voneinander löst, sondern dass ein Riss in der Außenschicht entsteht, der sich bis in die Innenschicht fortpflanzen kann. Im ungünstigsten Fall könnte der Schlauch dabei so beschädigt werden, dass ggf. ein Leck auftritt. Insbesondere bei niedrigen Temperaturen und einem unachtsamen Öffnen der Kringel kann es so zu Beschädigungen kommen, welche den Schlauch unbrauchbar machen könnten.

Angesichts der Nachteile, mit denen die im dargelegten Stand der Technik beschriebenen Ausführungsformen behaftet sind, ist es Aufgabe der Erfindung, die Sicherheit der Peelbarkeit eines verschweißten knickschädigungsfreien Schlauchkringels aus einem PVC-freien Mehrschichtschlauch für medizinische Zwecke zu verbessern.
Die miteinander verschweißten Kringel sollen einfach und sicher voneinander lösbar sein, insbesondere ohne Funktionsbeeinträchtigung. Zusätzlich soll der Mehrschichtschlauch auch ausreichend flexibel, elastisch und weich sein und trotzdem möglichst wenig knickanfällig, relative formstarr und thermisch stabil sein. Schließlich soll der Schlauch bei Berührung mit den in der Medizin üblichen Fluiden auch keine gefährlichen Substanzen in diese Fluide abgeben und überhaupt inert gegenüber medizinischen Lösungen sein.

Diese und weitere nicht näher genannte Aufgaben werden durch Verwendung einer Pufferschicht in dem PVC-freien Mehrschichtschlauch für medizinische Zwecke gemäß Anspruch 1 gelöst. Besondere Weiterentwicklungen werden in den von Anspruch 1 abhängigen Ansprüchen unter Schutz gestellt.

Gegenstand der Erfindung ist die Verwendung wenigstens einer Schicht als Pufferschicht C) in einem PVC-freien Mehrschichtschlauch für medizinische Zwecke zur Verbesserung der Sicherheit der Peelbarkeit eines verschweißten knickschädigungsfreien Schlauchkringels, aufweisend einen Durchmesser von größeren Durchmessern herab bis zu nur 50 mm Durchmesser, aus dem Mehrschichtschlauch,
wobei der Mehrschichtschlauch wenigstens drei Schichten umfasst, von denen eine Basisschicht A) aus einem ersten Kunststoffmaterial mit wenigstens einer Konnektionsschicht B) aus einem zweiten Kunststoffmaterial mittels wenigstens einer zwischen A) und B) angeordneten Pufferschicht C) aus einem dritten Kunststoffmaterial verbunden ist, dadurch gekennzeichnet, dass
sowohl das erste als auch das zweite Kunststoffmaterial jeweils 25 Gew.-% oder mehr als 25 Gew.-% bezogen auf das gesamte Gewicht der jeweiligen Schicht A) beziehungsweise B) aus Polyolefinen bestehen, während das dritte Kunststoffmaterial einen Anteil an thermoplastischem Elastomer, welches nicht Polyolefin ist, von mehr als 75 Gew.-% bezogen auf das Gesamtgewicht der Schicht C) besitzt.

Dadurch gelingt es auf überraschende Weise, die Sicherheit der Peelbarkeit des Schlauchkringels aus dem Mehrschichtschlauch zu verbessern.

Der vorliegenden Erfindung liegt mithin der Gedanke zugrunde, zwischen der Basis- oder Innenschicht eines Mehrschichtschlauches und der Konnektions- oder Außenschicht eine weitere Pufferschicht als Mittelschicht einzufügen. Durch den relativ höheren Elastomeranteil der Pufferschicht ist diese wesentlich weicher und mechanisch schwächer als die Basis- und Konnektionsschicht(en). Zwischen der Pufferschicht und der Innen- oder Außenschicht besteht damit auch eine geringere Haftung. Dies kann beim Öffnen der Kringel zwar dazu führen, dass die Außenschicht reißt, statt an der Verschweißstelle zu peelen. Der Riss führt aber regelmäßig nur bis zur Pufferschicht und setzt sich nie bis in die Innenschicht fort. Dabei werden entweder Außenschicht oder Puffer- und Außenschicht delaminiert. Weil die formgebende Innenschicht dabei nicht beschädigt wird, tritt das Problem von unbrauchbaren Schläuchen auch bei tieferen Temperaturen nicht mehr auf.

Weiterhin sind vorzugsweise die verschiedenen Kunststoffschichten im mehrschichtigen Schlauchmaterial so aufeinander abgestimmt, dass wenigstens eine Schicht als Basis- oder Innenschicht fungierend dem Schlauchmaterial eine genügende thermische Stabilität bei der Sterilisation verleiht, während wenigstens eine andere Schicht als Konnektions- oder Außenschicht fungierend, die Ausbildung einer festen und dichten Verbindung mit einem Beutel, Anschlussstutzen, Konnektor oder einem anderen Schlauch ermöglichen kann, ohne dass zusätzliche Haft-, Dichtungs- oder Versiegelungsmassen oder Hilfsmittel einzusetzen wären oder andere Versiegelungsverfahren (hochfrequente Energie oder ähnliche) anzuwenden wären.

Der Begriff der Peelbarkeit bzw. der verbesserten Sicherheit der Peelbarkeit spielt im Rahmen der Erfindung eine besondere Rolle für die Qualität der Mehrschichtschläuche. Dabei soll für die Erfindung unter "Peelbarkeit" oder "Peelfähigkeit" eine Eigenschaft verstanden werden, welche die Lösbarkeit von miteinander bei Schweißtemperaturen von 400 °C oder mehr peripher verschweißter Kringel eines Mehrschichtschlauches, der nach der Kringelbildung sterilisiert wurde, unter Einwirkung einer definierten Last beschreibt. Diese Last wird mit einem Fallgewicht aufgewendet. Hierbei wird ein Prüfhammer mit einem Gewicht von 5 kg an dem äußersten peripher verschweißten Kringel befestigt. Nach einem Freifall von 33 cm wird der verschweißte Kringel ruckartig belastet. Unter Einwirkung der Last werden die Kringel zwangsgeöffnet. Geschieht die Lösung benachbarter Kringel voneinander ohne Funktionsverlust des Schlauches, ist der Schlauch peelfähig.

Die erfindungsgemäß verwendete Pufferschicht C) im Mehrschichtschlauch ist wesentlich "elastischer" oder "weicher" als die Basisschicht A) und die Konnektionsschicht B). Dies wird durch den relativ hohen Elastomeranteil von größer 75 Gew.-%, vorzugsweise größer oder gleich 80 Gew.-% in der Schicht C) bewirkt. Besonders zweckmäßig ist der Anteil an thermoplastischem Elastomer in der Schicht C) gleich oder größer 90 Gew.-%, besonders bevorzugt besteht die Pufferschicht C) zu 100 Gew.-% aus thermoplastischem Elastomer, wobei die Mengenangaben sich jeweils auf das Gesamtgewicht der Schicht C) beziehen. Bei den thermoplastischen Elastomeren, welche für die Schicht C) einsetzbar sind, handelt es sich um solche polymeren Materialien, welche keine Polyolefine sind.

Polyolefine im in der vorliegenden Beschreibung gebrauchten Sinne sind im weitesten Sinne Polymere der Alkene. Hierzu gehören unter anderem Polyalkane. Beispiele umfassen insbesonders Polymethylen, Polyethylene wie PE-LD, PE-HD, PE-LLD, mPE-LLD, modifizierte Polyethylene wie PE-X, VPE oder XLPE, Polypropylene, Poly-1-buten und höhere Poly(alpha-olefin)e. Hierbei kann es sich um Homopolymere, Copolymere, sowohl als Block- als auch statistische Copolymere, und Terpolymere handeln. Besondere Bedeutung kommen den Polymeren und Copolymeren des Propylens bei. Hierzu wiederum zählen unter anderem Poly(propylene), die durch Übergangsmetall-Katalysatoren polymerisiert wurden, Metallocen-Polypropylene, ataktische und syndiotaktische Polypropylene und besonders bevorzugt Copolymere mit Ethylen. Hier wiederum sind solche Copolymere bevorzugt, die einen geringen Ethylengehalt aufweisen, beispielsweise von 5 % Ethylen oder weniger.

Im Gegensatz zur Pufferschicht C) in welcher der Polyolefingehalt auf weniger als 25 Gew.-% beschränkt ist, weisen sowohl das erste als auch das zweite Kunststoffmaterial jeweils 25 Gew.-% oder mehr als 25 Gew.-%, besonders zweckmäßig zu mehr als 30 Gew.-% bezogen auf das gesamte Gewicht der jeweiligen Schichten A) bzw. B) Polyolefine auf. Hierdurch werden sowohl die Basis- als auch die Konnektionsschicht weniger elastisch als die Pufferschicht, mechanisch aber stärker oder stabiler als die Pufferschicht.

Auch zwischen erstem und zweitem Kunststoffmaterial kann man im Hinblick auf die Härte respektive Weichheit der Materialien Unterschiede feststellen. So ist es für den Mehrschichtschlauch der Erfindung bevorzugt, dass das erste Kunststoffmaterial der Basisschicht ein relativ hartes Material ist. In einer besonderen Ausführungsform verfügt das erste Kunststoffmaterial über eine Härte Shore D von kleiner oder gleich 35. Besonders bevorzugt von kleiner oder gleich 32. Bei dem zweiten Kunststoffmaterial für die Konnektionsschicht handelt es sich zweckmäßigerweise um ein etwas weicheres Kunststoffmaterial, verglichen mit dem Kunststoffmaterial der ersten Schicht. Von besonderem Interesse für die Erfindung sind für das zweite Kunststoffmaterial solche Kunststoffmaterialien, die eine Härte Shore D kleiner oder gleich 60 aufweisen. Zweckmäßig sind Materialien mit Shore D kleiner oder gleich 55, besonders zweckmäßig kleiner oder gleich 50. In jedem Fall ist Shore D > 32 bevorzugt. Besonders bevorzugt ist Shore D des zweiten Kunststoffmaterials > 35.

Im Rahmen der Erfindung ist es außerdem besonders bevorzugt, dass das dritte Kunststoffmaterial einen Wert für die Härte Shore A aufweist, der kleiner ist als der Wert für die Härte Shore A des zweiten Kunststoffmaterials. Besonders vorteilhaft sind Kunststoffmaterialien für die Pufferschicht mit einem Wert für die Härte Shore A von < 60, insbesondere < 55, ganz besonders bevorzugt < 50.

Auch im Hinblick auf die Schmelztemperatur bzw. die Glastemperatur der verschiedenen Kunststoffmaterialien, wie sie in den verschiedenen Schichten des erfindungsgemäßen Mehrschichtschlauches zum Einsatz kommen, ergeben sich Unterschiede.

Weiterhin wird im Rahmen der Erfindung unter "Sterilisation" allgemein ein Verfahren für die Abtötung bzw. Inaktivierung (Viren) aller Mikroorganismen einschließlich der hochresistenten Dauerformen verstanden, wobei die erfindungsgemäßen Mehrschichtschläuche insbesondere einer Dampfsterilisation im Autoklaven mit gespanntem Wasserdampf von mindestens 121 °C, entsprechend etwa einer Atmosphäre Überdruck, der sogenannten Autoklavierung oder Autoklavierungsbehandlung widerstehen, ohne Schaden zu nehmen.

Daneben werden für die Erfindung unter "Kunststoffmaterial" solche Materialien verstanden, deren wesentliche Bestandteile aus makromolekularen organischen Verbindungen bestehen, wobei die Kunststoffmaterialien auch als Polymere bezeichnet werden, wozu insbesondere Homopolymere als auch Copolymere (statistische Copolymere, Block- und/oder Propfpolymere) sowie Mischungen (= Blends) aus den vorgenannten Stoffen gehören.

Ein Kriterium für die erfindungsgemäße Auswahl und Zuordnung eines Polymers zu einem Kunststoffmaterial und damit zu einer bestimmten Funktionsschicht des mehrschichtigen PVC-freien Schlauches für medizinische Zwecke kann auch die Formbeständigkeit während einer Hitzesterilisation darstellen.

Ein Kunststoffmaterial gilt in diesem Sinne als formbeständig, wenn eine Schlauchprobe von mindestens 10 mm Länge, einem Innendurchmesser von 5 mm und einem Außendurchmesser von 7 mm eine Heißdampfsterilisation bei 121 °C mit mindestens 15 min. Aufheizzeit, mindestens 15 min. Haltezeit und mindestens 10 min. Kühlzeit ohne optisch sichtbare Formveränderung "zusammenfallen" oder "Ovalität" etc. übersteht.

Die angegebenen Temperaturen beziehen sich dabei jeweils auf den Druck bei der Dampfsterilisation, also ca. eine Atmosphäre Überdruck. Es versteht sich jedoch, dass die Druckabhängigkeit der Erweichungstemperatur im betrachteten Bereich zwischen Normaldruck und dem zum Dampfsterilisieren nötigen Überdruck im Allgemeinen vernachlässigbar gering ist.

Je nach gewünschter Funktion des PVC-freien Mehrschichtschlauches kann es von Vorteil sein, eine speziell zur Verbindungsbildung befähigte Schicht B) nur außen oder sowohl außen als auch innen anzuordnen. Eine weitere Pufferschicht im Inneren (zwischen einer inneren Konnektionsschicht B) und der Basisschicht A)) ist optional.

In besonders zweckmäßiger Ausführungsform kennzeichnet sich der Mehrschichtschlauch durch eine Abfolge der Schichten A) C) B) oder B) A) C) B), jeweils von innen nach außen.
In jedem Fall kann ein Schlauchkringel aus dem Mehrschichtschlauch ohne Probleme gelöst werden. Im ersten Fall ist der Schlauch zum Einführen in ein Hohlteil gedacht, dessen Innenoberfläche aus geeignetem Material zur Ausbildung einer Verbindung besteht, während bei Anordnung zweier Verbindungsschichten (außen und innen) beim PVC-freien Mehrschichtschlauch die beiden genannten Verbindungsmöglichkeiten wahlweise oder gleichzeitig realisierbar sind.

Bei besonders zweckmäßigen PVC-freien Mehrschichtschläuchen mit verbesserter Sicherheit der Peelbarkeit ist das erste Kunststoffmaterial für die Innenschicht zum überwiegenden Teil ein synthetischer Kautschuk auf Isopren-Basis oder Polypropylen mit einer Dichte von 0,9 g/cm³ und das zweite Kunststoffmaterial für die Außenschicht zum überwiegenden Teil ein Polypropylen Copolymer mit einem geringen Ethylengehalt von etwa 4 %. Die erfindungsgemäß verwendete Pufferschicht dazwischen ist bevorzugt ein SEBS oder eine Mischung von SEBS Polymeren mit einem Styrolgehalt von 13 bis 30 %. Diese Kombination für jede Basisschicht A), Außenschicht B) und Pufferschicht C) kann bereits eine Vielzahl an geforderten Eigenschaften erfüllen. Für die Erfindung besonders vorteilhaft ist eine Polymerverwendung gemäß der folgenden Auflistung. Prozentangaben bedeuten jeweils Gewichtsprozent.

| | |
|---|---|
| Innenschicht: | Dicke: 900 - 980 µm |
| | bei ca. 1 mm Wandstärke des Schlauchs |
| | |
| Blend aus | |
| 50 - 75 % SIS | (HVS/3, Kuraray oder Hybrar 7125F, Kuraray) |
| und | |
| 50 - 25 % PP-R | (PP 23M10cs264, REXENE); |
| | - PP mit Shore D ≤ 32, p = 0,9 g/cm³; (z.B. Adflex 100 G, |
| | Himont, mit bis zu 50 % Kautschukanteil, z.B. PIB, Styrol-Ethylen-Butylen-Kautschuk, Styrol-Ethylen-Propylen-Kautschuk, SIS oder (RD204CF, Borealis A/S); |
| | - PP-Copolymer |
| Außenschicht: | Dicke 15 - 100 µm |
| | |
| Blend aus | |
| 50 - 75 % PP-R | (PP 23M10cs264, REXENE oder RD 204 CF, Borealis A/S) und |
| 25 - 50 % SIS | (HVS/3, Kuraray oder Hybrar 7125 F, Kuraray); |
| Pufferschicht: | Dicke 10 - 50 µm |
| | |
| 100 % SEBS | (Kraton 1657, Shell) SEBS mit 13 % Styrol oder |
| | (Tuftec H 1052, Asahi) SEBS mit 20 % Styrol oder |
| | (Tuftec H 1062, Asahi) SEBS mit 18 % Styrol |
| oder | |
| 100 % SBS | (Styroflex BX 6105, BASF) SBS mit 70 % Styrol |
| oder | |
| Blend aus | |
| 50 - 90 % SEBS | (z.B. Kraton G 1726, Shell) und |
| 10 - 50 % SEBS | (z.B. Kraton 1625M, Shell) SEBS mit 29 % Styrol; |

Die Abkürzungen bedeuten im Einzelnen:
- PP-R: = Polypropylen-Randomcopolymer
- SIS: = Styrol-Isopren-Styrol
- SEBS: = Styrol-Ethylen-Butylen-Styrol-Kautschuk
- SBS: = Styrol-Butylen-Styrol-Kautschuk

Wiederum kann im Hinblick auf die Haftung zwischen Schichten aus den Materialien A), B) und C) gesagt werden, dass diese grundsätzlich ausreichend ist. Sie kann jedoch in vorteilhafter Weise dadurch gesteigert werden, dass die Schichten A), B) und/oder C) jeweils zusätzlich bis zu 40 Gew.-%, bezogen auf 100 Gew.-% ihrer wie oben beschriebenen und definierten Zusammensetzung, desjenigen Kunststoffmaterials aufweisen, welches zur Ausbildung einer oder beider benachbarten Schichten des PVC-freien Mehrschichtschlauches dient.

Durch diese "Materialvermittlung" bzw. die Substitution von Material wird die Kompatibilität der miteinander zu einem Schlauch geformten Schichten deutlich gesteigert, ohne die sonstigen Eigenschaften in Frage zu stellen.

Die Kunststoffmaterialien des PVC-freien Mehrschichtschlauches sind für alle Schichten des Schlauchs so gewählt, dass sie im Wesentlichen aus Polyolefinhomopolymeren oder Polyolefincopolymeren und Abwandlungen davon (z.B. SEBS) bestehen. Der PVC-freie Mehrschichtschlauch besteht ausschließlich aus umweltverträglichen Materialien, die eine problemlose Verbindungsbildung mit Konnektoren bei der Dampfsterilisation gestatten und gleichzeitig alle weiteren Forderungen an einen medizinisch einsetzbaren Schlauch erfüllen, insbesondere also über eine verbesserte Peelbarkeit verfügen.

Hinsichtlich der Geometrie können die Schläuche selbst in allen erforderlichen und üblichen Dicken und Größen ausgeführt sein. Bevorzugt besteht der PVC-freie Mehrschichtschlauch zu mehr als 96 - 98 Vol.-%, bezogen auf das gesamte Volumen des Schlauchmaterials aus der Basisschicht A). In besonders zweckmäßiger Ausgestaltung ist der Mehrschichtschlauch dadurch gekennzeichnet, dass die Basisschicht A) eine Dicke im Bereich von 800 - 1000 µm aufweist. Von besonderem Interesse sind Basisschichten A), die eine Dicke von > 900 µm aufweisen, vorzugsweise von etwa 910 - 930 µm, besonders bevorzugt von 920 - 980 µm. Von besonderem Interesse für die Erfindung sind ebenso Ausführungsformen des Mehrschichtschlauches, bei denen die Außenschicht B) eine Dicke im Bereich von 15 - 100 µm aufweist. Besonders zweckmäßig sind Außenschichten B) respektive Konnektionsschichten B), welche eine Dicke im Bereich von 20 - 80 µm, vorzugsweise 30 - 70 µm und besonders bevorzugt von etwa 50 µm aufweisen. Ebenso sind Mehrschichtschläuche für die Erfindung von besonderem Interesse, bei denen die erfindungsgemäß verwendete Pufferschicht C) eine Dicke von weniger als 100 µm aufweist. In besonders vorteilhafter Abwandlung hiervon ist die erfindungsgemäß verwendete Pufferschicht C) dadurch gekennzeichnet, dass sie eine Dicke im Bereich von 10 - 30 µm, ganz besonders zweckmäßig eine Dicke im Bereich von etwa 20 - 30 µm aufweist. Besonders vorteilhaft sind insgesamt solche Ausführungsformen des Mehrschichtschlauches, bei denen die Dicke der erfindungsgemäß verwendeten Pufferschicht C) relativ zur Dicke der Außenschicht B) kleiner ist. Hierdurch wird ein sicheres Peelen von miteinander verschweißten Schlauchkringeln oder Bestandteilen des Schlauches möglich, ohne die empfindliche Innenschicht des Schlauches zu gefährden.

Von Bedeutung für die Erfindung ist die Auswahl der miteinander zu extrudierenden Schichtmaterialien, wobei besonders bevorzugt Kunststoffmaterialien oder Schichten so ausgewählt werden, dass alle Schichten des PVC-freien Mehrschichtschlauches im Wesentlichen aus Polyolefinhomopolymeren und/oder Polyolefincopolmeren bestehen, oder aus darauf basierenden Polymeren wie z.B. Abwandlungen von Polyoefinen (z.B. SEBS, SBS und dergleichen).

Die Koextrusion solcher Materialien ist zwar grundsätzlich bekannt, es ist jedoch aufgrund vorhandener Erfahrungen nicht ohne Weiteres die erfolgreiche Realisierung eines so komplexen Mehrschichtschlauches vorhersehbar gewesen. In der Praxis zeigte sich sonst immer wieder, dass selbst bei Zuhilfenahme von gegebenenfalls tabellierten Eigenschaften von Polymeren, etwa Daten zur Verbundhaftung, der Einsatz solcher Materialien nicht notwendigerweise zum Erfolg führt. So ist die Abstimmung der Viskosität der Schmelze bei der Coextrusion von Kautschuken wie SEBS mit PP besonders schwierig. Weiterhin ist es vorteilhaft möglich, dass man zur Formung eines PVC-freien Mehrschichtschlauches die Kunststoffmaterialien so auswählt, dass alle Schichten des Schlauches zusätzlich bis zu 40 Gew.-% des Materials der jeweils benachbarten Schicht oder Schichten enthalten können. Hierdurch kann in gewissem Maße eine geringe Haftung zwischen zwei benachbarten Schichten kompensiert werden. Nach der eigentlichen Formung kann der erhaltene Schlauch auf übliche Arten weiter bearbeitet werden. Bevorzugt wird er nach dem Formen mit Wasser schockgekühlt. Hierdurch wird wegen des "Einfrierens" des amorphen Zustandes ein optimaler Verbund mit hoher Flexibilität und ausreichender Steifigkeit erreicht, aber vor allem wird durch das schockartige Kühlen der Schmelze die Transparenz des Schlauches verbessert, weil sich keine kristallinen Bereiche ausbilden können. Dies führt zu einem geringen Kristallinitätsgrad und damit zur hohen Transparenz und Zähigkeit.

Der PVC-freie Mehrschichtschlauch eignet sich hervorragend für eine Verwendung im medizinischen Bereich. Die Materialien des Mehrschichtschlauches sind alle so ausgewählt, dass der Schlauch durchsichtig, knickbeständig und flexibel, insbesondere aber heißsterilisierbar und durch gleichzeitig vom Schlauch ausgeübte Presskraft mit einem entsprechenden Konnektor fest und bakteriensicher verbindbar ist. Zusätzlich ist der Non-PVC-Mehrschichtschlauch auch noch biokompatibel. Die Verwendung von PVC, welches immer einen Anteil an Weichmachern enthält, wird vermieden, ebenso werden keine Haftvermittler benötigt, die gegebenenfalls durch die Kunststoffmaterialschichten hindurch diffundieren könnten.

Aufgrund seiner hervorragenden Material- und Gebrauchseigenschaften wird der PVC-freie Mehrschichtschlauch mit besonders großem Vorteil als fluidführende Leitung in der Dialyse, Infusion oder künstlichen Ernährung verwendet. Zu diesem Zweck kann es vorteilhafterweise vorgesehen sein, dass zumindest der Anschlussbereich zum Anschluss an den Vorratsbeutel mit einer Schweißlippe versehen ist.

Hierbei macht man sich vor allem die Kompatibilität des Konnektionsschichtmaterials des Mehrschichtschlauches mit den Anschlussstücken von medizinischen Beuteln (vor allem aus Polypropylen) und/oder speziell in der Medizintechnik üblichen Verbindungstechniken, beispielsweise in Form von Konnektoren aus Polypropylen zu Nutze.

Konnektoren oder Beutel können dabei mit einer rauen Oberfläche versehen sein, auf die der PVC-freie Mehrschichtschlauch gesteckt wird, so dass die Innenoberfläche des PVC-freien Mehrschichtschlauches mit der Konnektionsschicht B) des Schlauches einen Presssitz mit der rauen Außenoberfläche des Konnektors oder Beutels (ggf. Anschlussstutzen des Beutels) bildet.

Durch die Oberfläche der Polypropylenteile sowie die Fließeigenschaft der Konnektionsschicht B) des Schlauches bei der Einwirkung von Hitze etwa bei der Dampfsterilisation ist durch Einfließen des Materials der Konnektionsschicht in die Unebenheiten der Oberfläche des Konnektors oder Beutelstutzens eine bereits gute und feste Verbindungsausbildung gegeben. Noch besser wird die Verbindung, wenn die zur Herstellung der Konnektionsschicht B) des Schlauches verwendeten Kunststoffmaterialien mit einem Anteil zwischen 1 und 40 Gew.-%, bezogen auf 100 Gew.-% des Materials der Konnektionsschicht mit einem Kunststoffmaterial, aus welchem der Konnektor oder der Anschlussstutzen des Beutels besteht, geblendet werden. Die Verbindung kann durch Aufrauhen der Oberfläche verbessert werden.

Weitere Vorteile und Einzelheiten der Erfindung ergeben sich aus den nachfolgenden Beispielen, die unter Bezugnahme auf die beigefügten Figuren erläutert werden.

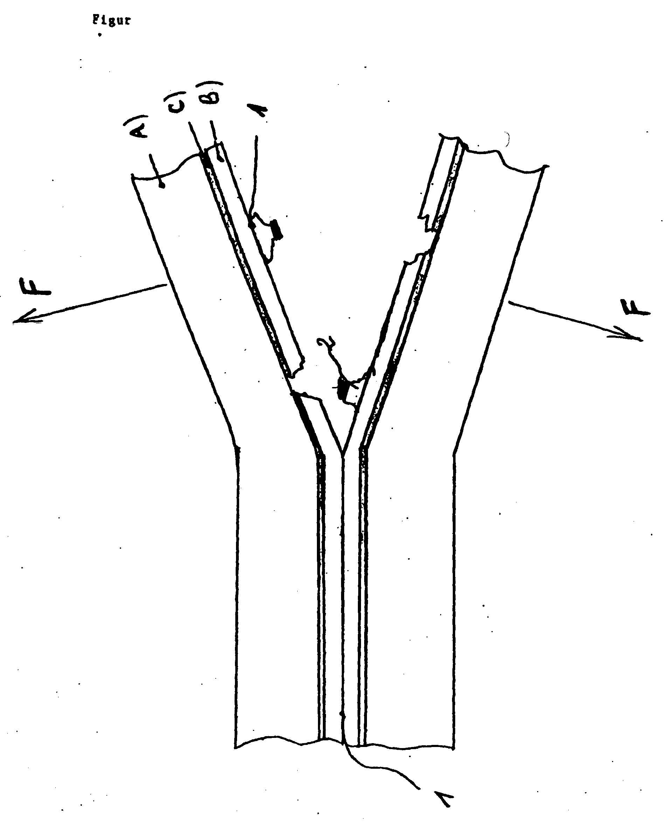

Die Figur zeigt eine Prinzipskizze eines PVC-freien Mehrschichtschlauches.

Bei der Darstellung gemäß der Figur handelt es sich um eine prinzipielle Darstellung eines Mehrschichtschlauches. Insbesondere wird in der Figur ein Teil eines Längsschnitts zweier miteinander verschweißter Abschnitte (Kringel) eines dreischichtigen Schlauches dargestellt, wobei im Längsschnitt Bereiche des Schlauches weggelassen wurden, die nicht an der Verschweißung zweier Schichten B) beteiligt sind.

In der Figur bezeichnet A) die Innenschicht eines Mehrschichtschlauches. B) steht für die Außenschicht eines Mehrschichtschlauches, während C) die erfindungsgemäß verwendete Zwischen- oder Pufferschicht beschreibt, welche zwischen den Schichten A) und B) angeordnet ist.
In der Figur werden zwei miteinander an der Oberfläche der Schicht B) verschweißte Abschnitte eines Mehrschichtschlauches gezeigt. Das Bezugszeichen 1 steht für die Schweißnaht, welche durch Verschweißen zweier Abschnitte der Außenschicht des Mehrschichtschlauches erhältlich ist. Man erkennt in der dargestellten Prinzipsskizze Bereiche, in welchen die Schweißnaht zwischen der Außenschicht B) des Mehrschichtschlauches geöffnet wurde, während in anderen Bereichen die Schweißnaht noch intakt ist, die zwei Abschnitte oder Kringel des Schlauches also noch miteinander verschweißt sind. Durch Anwendung einer Kraft F, die in der durch die Pfeilrichtung angedeuteten Weise auf die miteinander verschweißten Kringel des Mehrschichtschlauches einwirkt, kommt es zum Lösen der beiden miteinander verschweißten Schlauchkringel. Dabei bleibt der Innenschlauch A) vollständig intakt, während sich, wie bspw. an dem Bezugszeichen 2 dargestellt, für den Fall des Einreißens der Außenschicht B) spätestens im Bereich der Pufferschicht C) eine saubere, zerstörungsfreie Trennung zwischen den Schlauchkringeln ergibt. Mithin lässt sich selbst bei Einreißen der Außenschicht die Funktionsfähigkeit des Mehrschichtschlauches aufrecht erhalten. Der Schlauch lässt sich durch die erfindungsgemäß verwendete Pufferschicht sicher peelen.

Nachfolgend wird die Erfindung weiter anhand von Versuchen zur Peelfähigkeit von exemplarischen Mehrschichtschläuchen erläutert.

In der Tabelle 1 (nachfolgend) wird die Zusammensetzung der Schichten der Mehrschichtschläuche angegeben. In der Tabelle 1 bedeutet AS: Außenschicht, MS: Mittelschicht bzw. hier Pufferschicht und IS: Innenschicht. Alle in der ersten Spalte der Tabelle angegebenen Materialien sind, sofern es sich um Blends handelt, vorher compoundiert und granuliert. Es handelt sich nicht um Dryblends. Der MFI der Blends SA131 war 5,9 (als Außenschicht), der MFI des Blends SI07 war 15 (als Pufferschicht), der MFI von XM130 betrug 4,6 (als Innenschicht). Die Kringel wurden gemäß Stand der Technik hergestellt, danach bei den in den Tabellen 3 und 4 angegebenen Temperaturen verschweißt und nach üblichen Verfahren sterilisiert und dann vier Tage unter den in Tabellen 3 und 4 angegebenen Prüftemperaturen gelagert. Im Anschluss daran wurden die Kringel mit der eingangs beschriebenen Methode des Prüfhammers getestet. Das Fallgewicht des Prüfhammers betrug 5 kg, die Freifallhöhe des Hammers war 33 cm. Nach 33 cm Freifall des Prüfhammers werden die Kringel belastet und zwangsgeöffnet. Die Prüfung auf Lösbarkeit der Kringel von sterilisierten Schlauchkringeln wurde bei verschiedenen Temperaturen, nämlich 23 °C, 15 °C und 5 °C durchgeführt.

Die Ergebnisse der Untersuchungen sind in den nachfolgenden Tabellen wiedergegeben.

**Tabelle 1**

| **Versuchs-Nr.** | **(1.) V40/03** | | | **(2.) V41/03** | | | **(3.) V42/03** | | | **(4.) V43/03** | | | **(5.) V44/03** | | |
|---|---|---|---|---|---|---|---|---|---|---|---|---|---|---|---|
| | AS | MS (PS) | IS | AS | MS (PS) | IS | AS | MS (PS) | IS | AS | MS (PS) | IS | AS | MS (PS) | IS |
| **Material (Gew.%)** | 50µm | 30µm | 920µm | 50µm | 30µm | 920µm | 50µm | 30µm | 920µm | 50µm | 30µm | 920µm | 50µm | 30µm | 920µm |
| **RD204CF** Co-PP(ca.4%Ethylen), MF=8/230°C, Tm=151°C | 60 | | 32 | 60 | | 32 | 60 | | 32 | 60 | | 32 | 60 | | 32 |
| **Hybrar7125F** SIS(20%Styrol), MF=4/230°C, Tg-ps=90°C | 40 | | 68 | 40 | | .68 | 40 | | 68 | 40 | | 68 | 40 | | 68 |
| **KratonG1726** SEBS(30%Styrol) MF=65/?°C, Tg-ps=90°C | | 60 | | | | | | | | | | | | | |
| **Kraton1652M** SEBS(29%Styrol) MF=10/?°C, Tg-ps=90°C | | 40 | | | | | | | | | | | | | |
| **Kraton1657** SEBS(13%Styrol) MF=8/?°C, Tg-ps=90°C | | | | | 100 | | | | | | | | | | |
| **TuftecH1052** SEBS(20%Styrol) MF=13/230°C, Tg-p=90°C | | | | | | | | 100 | | | | | | | |
| **TuftecH1062** SEBS(18%Styrol) MF=4,5/230°C, Tg-ps=90°C | | | | | | | | | | | 100 | | | | |
| **StyroflexBX6105** SBS(70%Styrol) MF=-12,5/230°C, Tg-p=90°C | | | | | | | | | | | | | | 100 | |
| **Bemerkungen** | SA131 | S107 | XM130 | SA131 | ···· | XM130 | SA131 | ···· | XM130 | SA131 | ···· | XM130 | SA131 | ···· | XM130 |

**Tabelle 2**

| **1. Hersteller der Materialien:** | | | | | | | |
|---|---|---|---|---|---|---|---|
| | | | | | | | |

| **Handelsname** | | **Material** | | | **Hersteller** | | |
|---|---|---|---|---|---|---|---|
| RD204CF | | PP-Copolemer | | | Borealis A/S | | |
| Hybrar 7125F | | SIS-Elastomer | | | Kuraray Co., Ltd. | | |
| KratonG1726 | | SEBS-Elastomer | | | Shell Chemical Company | | |
| Kraton1652M | | SEBS-Elastomer | | | | | |
| Kraton1657 | | SEBS-Elastomer | | | | | |
| TuftecH1052 | | SEBS-Elastomer | | | Asahi Chemical Industry Co., Ltd. | | |
| TuftecH1062 | | SEBS-Elastomer | | | | | |
| | | | | | | | |

| **2. Einige Eigenschaften der Materialien** | | | | | | | |
|---|---|---|---|---|---|---|---|
| | | | | | | | |

| **Handelsname** | **Gruppe** | | **Ethylen bzw. Styrol** | **Tm bzw. Tg** | | **MFI** | **Shore A** |
|---|---|---|---|---|---|---|---|
| | | | **[%]** | **[°C]** | | **[g/10min]** | |
| RD204CF | I | | 4 | 151 | | 8 | viel härter! |
| Hybrar7125F | II | | 20 | 90 u. -15 | | 4 | 64 |
| KratonG1726 | | | 30 | 90 u. -45 | | 65 | 60 |
| Kraton1652M | | | 29 | 90 u. -45 | | 10 | 75r |
| Kraton1657 | | | 13 | 90 u. -55 | | 8 | 65 |
| TuftecH1052 | | | 20 | 90 u. -26 | | 13 | 67 |
| TuftecH1062 | | | 18 | 90 u. -32 | | 4,5 | 67 |

**Tabelle 3**

| **PrüfTemperatur** | | **Sterile Musterschläuche** | | | | | |
|---|---|---|---|---|---|---|---|
| | **Fehlerart** | Schweißtemperaturstufe I | | | | | |
| | | Standard* | V40 | V41 | V42 | V43 | V44* |
| | Schweißtmp. | 400°C | 410°C | 410°C | 410°C | 410°C | 430°C |
| **5°C** (je 15 Coils) | Komplettriss (>10mm, Ruptur) | 0 | 0 | 0 | 0 | 0 | 0 |
| | Durchriss (≥1mm, Loch!) | 1 (7%) | 0 | 0 | 0 | 0 | 0 |
| | Starkriss (300µ∼<1mm) | 3 (20%) | 0 | 0 | 0 | 0 | 0 |
| | Leichtriss (100µ∼300µ) | 8 (53%) | 0 | 0 | 0 | 0 | 0 |
| | Peelen/ Delaminieren | 3 (20%) | 15 (100%) | 15 (100%) | 15 (100%) | 15 (100%) | 15 (100%) |
| **16°C** (je 15 Coils) | Komplettriss (>10mm, Ruptur) | 0 | 0 | 0 | 0 | 0 | 0 |
| | Durchriss (≥1mm, Locht) | 2 (13%) | 0 | 0 | 0 | 0 | 0 |
| | Starkriss (300µ∼<1mm) | 3 (20%) | 0 | 0 | 0 | 0 | 0 |
| | Leichtriss (100µ-300µ) | 3 (20%) | 0 | 0 | 0 | 0 | 0 |
| | Peelen/ Delaminieren | 7 (47%) | 15 (100%) | 15 (100%) | 15 (100%) | 15 (100%) | 15 (100%) |
| **22 °C** (je 15 Coils) | Komplettriss (>10mm, Ruptur) | 0 | 0 | 0 | 0 | 0 | 0 |
| | Durchriss (≥1mm, Loch!) | 0 | 0 | 0 | 0 | 0 | 0 |
| | Starkriss (300µ∼<1mm) | 0 | 0 | 0 | 0 | 0 | 0 |
| | Leichtriss (100µ∼300µ) | 9 (60%) | 0 | 0 | 0 | 0 | 0 |
| | Peelen/ Delaminieren | 6 (40%) | 15 (100%) | 15 (100%) | 15 (100%) | 15 (100%) | 15 (100%) |

| | | | | | | | |
|---|---|---|---|---|---|---|---|
| * Standard bezieht sich auf den Schlauch ohne Pufferschicht | | | | | | | |

**Tabelle 4**

| **PrüfTemperatur** | | **Sterile Musterschläuche** | | | | | |
|---|---|---|---|---|---|---|---|
| | **Fehlerart** | Schweißtemperaturstufe II | | | | | |
| | | Standard* | V40 | V41 | V42 | V43 | V44* |
| | Schweißtmp. | 430°C | 440°C | 440°C | 440°C | 440°C | 460°C |
| **5°C** (je 15 Coils) | Komplettriss (>10mm, Ruptur) | 0 | 0 | 0 | 0 | 0 | 0 |
| | Durchriss (≥1mm, Loch!) | 1 (7%) | 0 | 0 | 0 | 0 | 0 |
| | Starkriss (300µ∼<1mm) | 7 (47%) | 0 | 0 | 0 | 0 | 0 |
| | Leichtriss (100µ∼300µ) | 5 (33%) | 0 | 0 | 0 | 0 | 0 |
| | Peelen/ Delaminieren | 2(13%) | 15 (100%) | 15 (100%) | 15 (100%) | 15 (100%) | 15 (100%) |
| **16°C** (je 15 Coils) | Komplettriss (>10mm, Ruptur) | 0 | 0 | 0 | 0 | 0 | 0 |
| | Durchriss (≥1mm, Loch!) | 1 (7%) | 0 | 0 | 0 | 0 | 0 |
| | Starkriss (300µ∼<1mm) | 2 (13%) | 0 | 0 | 0 | 0 | 0 |
| | Leichtriss (100µ∼300µ) | 2 (13%) | 0 | 0 | 0 | 0 | 0 |
| | Peelen/ Delaminieren | 10 (67%) | 15 (100%) | 15 (100%) | 15 (100%) | 15 (100%) | 15 (100%) |
| **22 °C** (je 15 Coils) | Komplettriss (>10mm, Ruptur) | 0 | 0 | 0 | 0 | 0 | 0 |
| | Durchriss (≥1mm, Loch!) | 0 | 0 | 0 | 0 | 0 | 0 |
| | Starkriss (300µ∼<1mm) | 6 (40%) | 0 | 0 | 0 | 0 | 0 |
| | Leichtriss (100µ∼300µ) | 3 (20%) | 0 | 0 | 0 | 0 | 0 |
| | Peelen/ Delaminieren | 6 (40%) | 15 (100%) | 15 (100%) | 15 (100%) | 15 (100%) | 15 (100%) |

| | | | | | | | |
|---|---|---|---|---|---|---|---|
| * Standard = ohne Pufferschicht | | | | | | | |

## Patentansprüche

1. Verwendung wenigstens einer Schicht als Pufferschicht C) in einem PVC-freien Mehrschichtschlauch für medizinische Zwecke zur Verbesserung der Sicherheit der Peelbarkeit eines verschweißten knickschädigungsfreien Schlauchkringels, aufweisend einen Durchmesser von größeren Durchmessern herab bis zu nur 50 mm Durchmesser, aus dem Mehrschichtschlauch,
wobei der Mehrschichtschlauch wenigstens drei Schichten umfasst, von denen eine Basisschicht A) aus einem ersten Kunststoffmaterial mit wenigstens einer Konnektionsschicht B) aus einem zweiten Kunststoffmaterial mittels wenigstens einer zwischen A) und B) angeordneten Pufferschicht C) aus einem dritten Kunststoffmaterial verbunden ist, **dadurch gekennzeichnet, dass**
sowohl das erste als auch das zweite Kunststoffmaterial jeweils 25 Gew.-% oder mehr als 25 Gew.-% bezogen auf das gesamte Gewicht der jeweiligen Schicht A) beziehungsweise B) aus Polyolefinen bestehen, während das dritte Kunststoffmaterial einen Anteil an thermoplastischem Elastomer, welches nicht Polyolefin ist, von mehr als 75 Gew.% bezogen auf das Gesamtgewicht der Schicht C) besitzt.

2. Verwendung nach Anspruch 1, **dadurch gekennzeichnet, dass**
das erste Kunststoffmaterial bei Temperaturen von mehr als 121 °C ohne Formschädigung hitzesterilisierbar ist, so dass die Basisschicht A) bei Temperaturen ≥ 121 °C noch formbeständig ist.

3. Verwendung nach Anspruch 1 oder 2, **dadurch gekennzeichnet, dass** das erste und/oder das zweite Kunststoffmaterial jeweils zu 30 Gew.-% oder mehr und besonders bevorzugt zu 50 Gew.-% oder mehr jeweils bezogen auf das gesamte Gewicht der jeweiligen Schicht A) beziehungsweise B) aus Polyolefinen bestehen.

4. Verwendung nach einem der vorhergehenden Ansprüche, **dadurch gekennzeichnet, dass** das erste Kunststoffmaterial und zweite Kunststoffmaterial jeweils zum überwiegenden Teil ein Polypropylen mit einer Dichte ρ ≤ 0,9 g/cm³ beziehungsweise ein synthetischer Kautschuk auf Isopren-Basis, zweckmäßig ein SIS, sind.

5. Verwendung nach einem der vorhergehenden Ansprüche, **dadurch gekennzeichnet, dass** das dritte Kunststoffmaterial einen Anteil an thermoplastischem Elastomer, welches nicht Polyolefin ist, von größer als 80 Gew.-%, besonders zweckmäßig von 100 Gew.-%, bezogen auf das Gesamtgewicht der Schicht C) besitzt.

6. Verwendung nach einem der vorhergehenden Ansprüche, **dadurch gekennzeichnet, dass** das dritte Kunststoffmaterial zum überwiegenden Teil ein SEBS oder SBS Kautschuk ist.

7. Verwendung nach einem der vorhergehenden Ansprüche, **dadurch gekennzeichnet, dass** das erste Kunststoffmaterial eine Härte Shore D ≤ 35, vorzugsweise eine Härte Shore D ≤ 32, hat.

8. Verwendung nach einem der vorhergehenden Ansprüche, **dadurch gekennzeichnet, dass** das zweite Kunststoffmaterial eine Härte Shore D ≤ 60, vorzugsweise eine Härte Shore D ≤ 65, besonders bevorzugt eine Härte Shore D ≤ 55 aufweist.

9. Verwendung nach einem der vorhergehenden Ansprüche, **dadurch gekennzeichnet, dass** das dritte Kunststoffmaterial einen Wert für die Härte Shore A aufweist, der größer ist als der Wert für die Härte Shore A des zweiten Kunststoffmaterials.

10. Verwendung nach einem der vorhergehenden Ansprüche, **dadurch gekennzeichnet, dass** die Basisschicht A) eine Dicke im Bereich von 800 bis 1000 µm aufweist.

11. Verwendung nach einem der vorhergehenden Ansprüche, **dadurch gekennzeichnet, dass** die Konnektionsschicht B) eine Dicke im Bereich vom 15 bis 100 µm aufweist.

12. Verwendung nach einem der vorhergehenden Ansprüche, **dadurch gekennzeichnet, dass** die Pufferschicht C) eine Dicke von weniger als 100 µm aufweist.

13. Verwendung nach einem der vorhergehenden Ansprüche, **dadurch gekennzeichnet, dass** die Pufferschicht C) eine Dicke im Bereich von 10 bis 30 µm aufweist.

14. Verwendung nach einem der vorhergehenden Ansprüche, **dadurch gekennzeichnet, dass** die Dicke der Pufferschicht C) geringer ist als die Dicke der Konnektionsschicht B).

15. Verwendung nach einem der vorhergehenden Ansprüche, **gekennzeichnet durch** eine Abfolge der Schichten A) C) B) oder B) A) C) B), jeweils von innen nach außen.

## Claims

1. Use of at least one layer as a buffer layer C) in a PVC-free multilayer tube for medical purposes for improving the reliability of the peelability of a welded tube without any kinking damage, having a diameter from larger diameters down to only 50-mm diameter from the multilayer tubing,
wherein the multilayer tubing comprises at least three layers, a base layer A), which is made of a first plastic material and is joined to at least one connection layer B) made of a second plastic material by means of at least one buffer layer C), which is made of a third plastic material and is arranged between A) and B), **characterized in that**
the first and second plastic materials each consist of polyolefins in the amount of 25 wt% or more than 25 wt%, based on the total weight of the respective layer A) and/or B), while the third plastic material contains a thermoplastic elastomer that is not polyolefin in amount of more than 75 wt%, based on the total weight of layer C).

2. Use according to Claim 1, **characterized in that** the first plastic material is heat-sterilizable at temperatures of more than 121°C without any damage to its shape, so that the base layer A) still has dimensional stability at temperatures ≥ 121°C.

3. Use according to Claim 1 or 2, **characterized in that** the first and/or second plastic material each consists of polyolefin in the amount of 30 wt% or more and especially preferably 50 wt% or more, each based on the total weight of the respective layer A) and/or B).

4. Use according to any one of the preceding claims, **characterized in that** the first plastic material and the second plastic material each comprise predominantly a polypropylene with a density ρ of ≤ 0.9 g/cm³ and/or a synthetic rubber based on isoprene, advantageously an SIS.

5. Use according to any one of the preceding claims, **characterized in that** the third plastic material contains a thermoplastic elastomer that is not polyolefin in amount greater than 80 wt%, especially preferably 100 wt%, based on the total weight of layer C).

6. Use according to any one of the preceding claims, **characterized in that** the third plastic material consists predominantly of an SEBS or SBS rubber.

7. Use according to any one of the preceding claims, **characterized in that** the first plastic material has a Shore D hardness of ≤ 35, preferably a Shore D hardness of ≤ 32.

8. Use according to any one of the preceding claims, **characterized in that** the second plastic material has a Shore D hardness of ≤ 60, preferably a Shore D hardness of ≤ 65, especially preferably a Shore D hardness of ≤ 55.

9. Use according to any one of the preceding claims, **characterized in that** the third plastic material has a value for the Shore A hardness which is greater than the value for the Shore A hardness of the second plastic material.

10. Use according to any one of the preceding claims, **characterized in that** the base layer A) has a thickness in the range of 800 µm to 1000 µm.

11. Use according to any one of the preceding claims, **characterized in that** the connection layer B) has a thickness in the range of 15 µm to 100 µm.

12. Use according to any one of the preceding claims, **characterized in that** the buffer layer C) has a thickness of less than 100 µm.

13. Use according to any one of the preceding claims, **characterized in that** the buffer layer C) has a thickness in the range of 10 µm to 30 µm.

14. Use according to any one of the preceding claims, **characterized in that** the thickness of the buffer layer C) is less than the thickness of the connection layer B).

15. Use according to any one of the preceding claims, **characterized by** a sequence of layers A), C), B) or B), A), C), B), each from the inside to the outside.

## Revendications

1. Utilisation d'au moins une couche en tant que couche tampon C) dans un tuyau multicouche exempt de PVC à finalité médicale, pour ainsi améliorer la sécurité en ce qui concerne la pelabilité d'une boucle de tuyau soudée laquelle peut subir un flambage sans être endommagée et laquelle présente un diamètre compris entre des diamètres relativement grands et un diamètre de seulement 50 mm et laquelle est réalisée en ledit tuyau multicouche,
ledit tuyau multicouche comprenant au moins trois couches dont une couche de base A), laquelle est réalisée en une première matière plastique, est reliée à au moins une couche de connexion B) réalisée en une deuxième matière plastique, cette liaison étant effectuée au moyen d'au moins une couche tampon C) qui est disposée entre A) et B) et qui est réalisée en une troisième matière plastique, **caractérisée en ce que**
la première et la deuxième matière plastique sont chacune constituée de polyoléfines à 25 % en poids ou à plus de 25 % en poids, par rapport au poids total de la couche A) ou B) concernée, alors que la troisième matière plastique possède une proportion d'un élastomère thermoplastique, ne agissant alors pas d'une polyoléfine, supérieure à 75 % en poids, par rapport au poids total de la couche C).

2. Utilisation selon la revendication 1, **caractérisée en ce que**
la première matière plastique peut être thermiquement stérilisée à des températures supérieures à 121 °C sans subir de déformations, faisant en sorte que la couche de base A) conserve sa forme à des températures ≥ 121 °C.

3. Utilisation selon les revendications 1 ou 2, **caractérisée en ce que** la première et/ou la deuxième matière plastique sont chacune constituée de polyoléfines à au moins 30 % en poids, et avec une préférence particulière à au moins 50 % en poids, par rapport au poids total de la couche A) ou B) concernée.

4. Utilisation selon l'une des revendications précédentes, **caractérisée en ce que** la première matière plastique et la deuxième matière plastique correspondant chacune majoritairement à un polypropylène ayant une densité p ≤ 0,9 g/cm³ et sinon à un caoutchouc synthétique à base d'isoprène, avantageusement de type SIS.

5. Utilisation selon l'une des revendications précédentes, **caractérisée en ce que** la troisième matière plastique possède une proportion d'un élastomère thermoplastique, ne agissant alors pas d'une polyoléfine, supérieure à 80 % en poids, de manière particulièrement avantageuse supérieure à 100 % en poids, par rapport au poids total de la couche C).

6. Utilisation selon l'une des revendications précédentes, **caractérisée en ce que** la troisième matière plastique correspond majoritairement à un caoutchouc de type SEBS ou SBS.

7. Utilisation selon l'une des revendications précédentes, **caractérisée en ce que** la première matière plastique présente une dureté Shore D ≤ 35, de préférence une dureté Shore D ≤ 32.

8. Utilisation selon l'une des revendications précédentes, **caractérisée en ce que** la deuxième matière plastique présente une dureté Shore D ≤ 60, de préférence une dureté Shore D ≤ 65 et, avec une préférence particulière, une dureté Shore D ≤ 55.

9. Utilisation selon l'une des revendications précédentes, **caractérisée en ce que** la troisième matière plastique présente, en termes de dureté Shore A, une valeur supérieure à la valeur que présente la deuxième matière plastique en termes de dureté Shore A.

10. Utilisation selon l'une des revendications précédentes, **caractérisée en ce que** la couche de base A) présente une épaisseur comprise entre 800 et 1000 µm.

11. Utilisation selon l'une des revendications précédentes, **caractérisée en ce que** la couche de connexion B) présente une épaisseur comprise entre 15 et 100 µm.

12. Utilisation selon l'une des revendications précédentes, **caractérisée en ce que** la couche tampon C) présente une épaisseur inférieure à 100 µm.

13. Utilisation selon l'une des revendications précédentes, **caractérisée en ce que** la couche tampon C) présente une épaisseur comprise entre 10 et 30 µm.

14. Utilisation selon l'une des revendications précédentes, **caractérisée en ce que** l'épaisseur de la couche tampon C) est inférieure à l'épaisseur de la couche de connexion B).

15. Utilisation selon l'une des revendications précédentes, **caractérisée par** une succession de couches de type A) C) B) ou B) A) C) B), toujours de l'intérieur vers l'extérieur.
